# EUROPEAN PATENT SPECIFICATION

(11) **EP 2 878 362 B1**
(45) Date of publication and mention of the grant of the patent: **04.07.2018**
(21) Application number: 13195276.4
(22) Date of filing: 02.12.2013
(51) Int. Cl.: B01D 63/02, B01D 65/00

(54) **CAPILLARY DIALYZERS**
HOHLFASERDIALYSATOREN
DIALYSEURS CAPILLAIRES

(43) Date of publication of application: 03.06.2015
(73) Proprietor: Gambro Lundia AB, 220 10 Lund (SE)
(72) Inventor: Wagner, Steffen, 72469 Meßstetten (DE); Hertzler, Bernd, 72336 Balingen (DE); Ermantraut, Stefan, 72336 Balingen (DE); Blickle, Rainer, 72475 Bitz (DE); Beck, Christof, 72475 Bitz (DE)
(74) Representative: Perchenek, Nils

(56) References cited:
- EP-A1- 1 570 896
- EP-A2- 0 844 015
- EP-A2- 1 323 462
- WO-A1-97/26030
- GB-A- 1 537 414
- JP-A- H0 549 875
- JP-A- 2010 051 844
- US-A- 4 686 039

## Description

### Technical Field

The present invention relates to capillary dialyzers for blood purification, methods for their production and housings for the capillary dialyzers.

### Background of the Invention

Capillary dialyzers are widely used for blood purification in patients suffering from renal insufficiency, i.e., for treatment of the patients by hemodialysis, hemodiafiltration or hemofiltration. A multitude of different models of capillary dialyzers is commercially available.

The devices generally consist of a housing comprising a tubular section with end caps capping the mouths of the tubular section. A bundle of hollow fiber membranes is arranged in the housing in a way that a seal is provided between a first flow space formed by the fiber cavities and a second flow space surrounding the membranes on the outside. The seal generally is provided by end wall means within the housing formed by a polymer mass in which the ends of the hollow fiber membranes are embedded. Examples of such devices are disclosed in EP 0 844 015 A2, EP 0 305 687 A1, and WO 01/60477 A2.

It is important that the seal between the flow space formed by the fiber cavities and the flow space surrounding the membranes on the outside, i.e., the blood compartment and the dialysate compartment of the dialyzer, remains intact at all times, especially during operation of the dialyzer in the treatment of a patient. Often, additional parts like sealing rings, gaskets, and support rings are provided in the dialyzer as additional safeguards against leakage. Production, handling, and subsequent assembly of the additional elements add to the complexity of the production process and the manufacturing cost. It would therefore be desirable to dispense with these additional parts in the dialyzer.

To this end, it is imperative that a tight connection between the end walls and the inner surface of the dialyzer housing is established and any delamination is prevented. The end walls and the dialyzer housing generally are comprised of different materials which also usually have different thermal expansion coefficients. As a consequence, temperature changes during manufacture or processing of the dialyzer, e.g. thermal sterilization, or pressure changes within the dialyzer during operation generate strain at the interface between the inner wall of the housing and the end wall means which may cause delamination of the end wall means from the housing and generate leaks. This is particularly the case for combinations of materials where the adhesive force between the respective materials is low, for instance, when a polypropylene housing is combined with polyurethane end wall means.

GB 1 537 415 A discloses a tubular membrane separation device, e.g. for hemodialysis, comprising a bundle of fibers enclosed in a casing. The housing has an annular chamber at each end having a number of circumferentially spaced passageways leading to the space surrounding the bundle and suitably provided by crennelations in the inner wall of said annular chamber.

EP 1 323 462 A2 discloses a filter device comprising an additional ring component. The ring provides an interface inside the filter which enables the potting compound to adhere to the filter and create a seal between a first and second fluid compartment within the filter. An embedded region of the ring possesses a detailed geometry which helps ensure that a delamination would be localized and unable to propagate from the first to the second compartment.

US 4 686 039 A discloses a fluid separation module having tube sheets positioned in the end portions of the cylindrical shell forming the exterior of the separation module. The end portions of the cylindrical shell have a plurality of spaced cuts extending longitudinally from the end of the shell to points spaced from the end of the shell, with the cuts dividing the end of the shell into a plurality of tabs.

### Summary of the Invention

The present application relates to a capillary dialyzer with a housing comprising a plurality of circumferential ridges in the zones where the end wall means which seal the individual compartments of the dialyzer from each other are located. The circumferential ridges provide additional anchors for the end wall means and prevent delamination of the end wall means from the inner surface of the housing.

The invention is defined in the appended claims.

### Short Description of the Drawings

- Fig. 1: shows two longitudinal-sectional partial views and a top view of an embodiment of the dialyzer housing of the present application;
- Fig. 2: shows a longitudinal-sectional schematic view of an embodiment of the capillary dialyzer of the present application.

### Detailed Description

A dialyzer housing 4 is provided which comprises a cylindrical first section 1 having an inner diameter d₁; a cylindrical second section 2 coaxial with the first section 1 and having an inner diameter d₂ which is larger than d₁; and a third section 3 joining the first section 1 and the second section 2, wherein a plurality of circumferential ridges 5 having a tooth profile and pointing in the direction of the axis of the first section 1 away from the first section 1 are provided on the inner surface of the third section 3.

In one embodiment, the inner diameter d₁ of the first section 1 is in the range of from 20 mm to 55 mm, for instance 25 to 50 mm. The difference of the diameters d₁ and d₂ generally is in the range of from 5 mm to 15 mm. In one embodiment of the dialyzer housing 4, the height of the third section 3 is in the range of from 0.36*(d₂-d₁) to 2.75*(d₂-d₁).

In the dialyzer housing 4, the faces of the circumferential ridges 5 form an angle in the range of from 20° to 40°. The circumferential ridges 5 have an elevation relative to the inner wall surface of the third section 3, measured from the top of the ridge in axial direction, in the range of from 1 to 10 mm. In one embodiment of the dialyzer housing 4, the number of circumferential ridges 5 is 2 to 5. In one embodiment of the dialyzer housing 4, the spacing of the tops of the individual circumferential ridges 5 is in the range of from 0.5 to 3 mm.

In the dialyzer housing 4, a plurality of openings 6 tapering from the outside towards the inside is provided in the wall of the second section 2. In one embodiment of the dialyzer housing 4, the openings 6 have a diameter on the outside surface of the second section 2 in the range of from 1 to 4 mm.

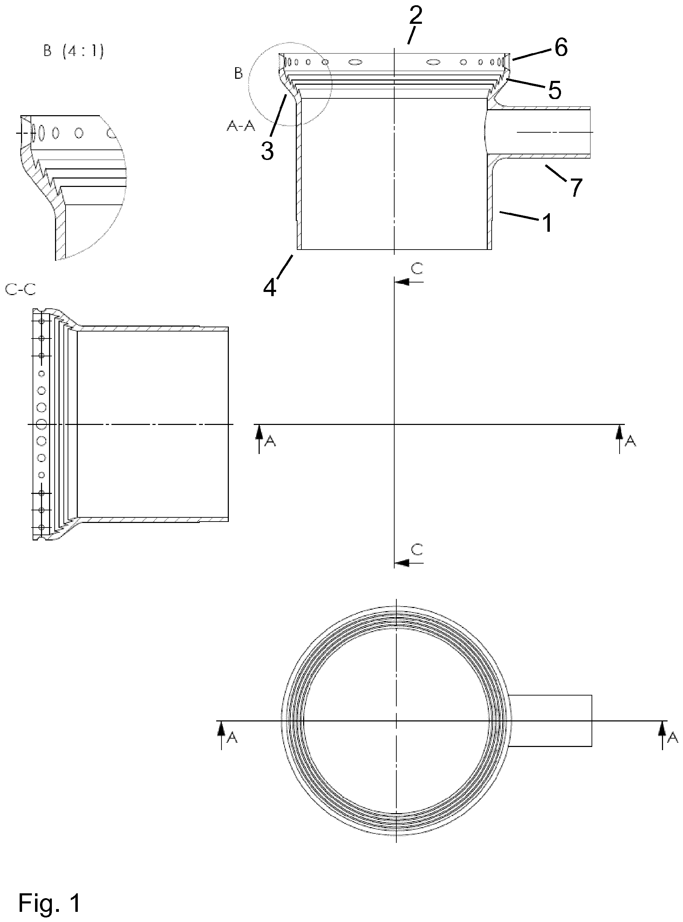

In one embodiment of the dialyzer housing 4, the first section 1 comprises fluid ports 7. Fig. 1 shows different (partial) views of an exemplary embodiment of the housing 4 featuring four circumferential ridges 5 having a tooth profile (see, e.g., Detail B) and featuring a plurality of conical holes in the second section 2. In this embodiment, the faces of the circumferential ridges 5 form an angle of 30°; and the spacing of the tops of the individual circumferential ridges 5 is 1 mm.

In one embodiment, the dialyzer housing 4 is comprised of a polyolefin. In one embodiment, the polyolefin is polypropylene.

The present application also provides a capillary dialyzer comprising a housing 4 as described above and second sections 2 and third sections 3 as described above on each end of the first section 1. The housing 4 defines a longitudinally extending internal chamber including a first end and a second end. A bundle 8 of semi-permeable hollow fiber membranes is disposed within the internal chamber and extends longitudinally from the first end of the internal chamber to the second end of the internal chamber. The hollow fiber membranes each have an outer surface and a first end and a second end corresponding to the first end and the second end of the internal chamber. End wall means 9 supporting the first and second ends of the hollow fiber membranes are provided within the internal chamber so as to sealingly separate the first ends and second ends of the hollow fiber membranes from the outer surfaces of the hollow fiber membranes between the first ends and second ends thereof. The end wall means fill the third sections 3.

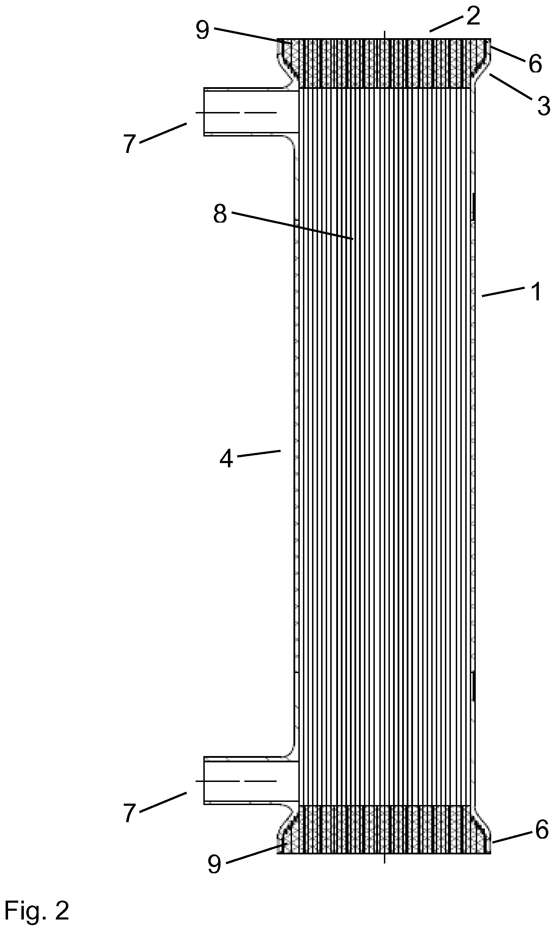

Each of the second sections 2 features a plurality of openings 6 in its wall, tapering from the outside towards the inside of the wall; and the end wall means 9 also fill the openings 6 of the second sections 2. A longitudinal-sectional schematic view of such a dialyzer is shown in Fig. 2. End wall means 9 extend over both second sections 2 and third sections 3 and also fill the conical holes 6 in the wall of the second sections 2.

The present disclosure also relates to processes for producing the dialyzer of the invention. An exemplary process comprises the steps of providing a housing 4 as described above which comprises second sections 2 and third sections 3 on each end of the first section 1. The housing 4 defines a longitudinally extending internal chamber including a first end and a second end. A bundle 8 of semi-permeable hollow fiber membranes having an outer surface, a first end and a second end is introduced into the internal chamber so that the bundle 8 extends longitudinally from the first end of the internal chamber to the second end of the internal chamber and the first ends and second ends of the hollow fiber membranes correspond to the first end and the second end of the internal chamber, respectively. End wall means 9 supporting the first and second ends of the hollow fiber membranes are provided within the internal chamber so as to sealingly separate the first ends and second ends of the hollow fiber membranes from the outer surface of the hollow fiber membranes between the first ends and second ends thereof, by introducing a potting material into the third sections 3 and second sections 2 of the housing 4 and filling the third sections 3 and the second sections 2 inclusive of the openings 6 in the wall of the second sections 2 with the potting material; and allowing the potting material to cure.

The end wall means 9 supporting the first and second ends of the hollow fiber membranes within the internal chamber are generated by potting the ends of the fiber with a polymer. A suitable potting material for the hollow fiber membranes is polyurethane. In an exemplary process, the ends of the housing 4 are closed and potting material, for instance polyurethane, is introduced into the housing 4 via one or both of the dialysis fluid ports 7. The potting material is distributed within the housing 4 of the dialyzer by centrifugation, i.e., rotating the dialyzer at high speed perpendicular to its longitudinal axis. The potting material is allowed to cure; thereby forming end wall means 9 on both ends of the bundle 8 of hollow fiber membranes.

The potting material shrinks during the curing process. The shrinkage of the cross-section of the end wall means 9 generates a centripetal tensile force on the circumferential ridges 5 and the openings 6 in the wall of the second sections 2. This provides an additional lock between the end wall means 9 and the housing 4. It prevents detachment of the end wall means 9 from the inner wall of the housing 4 and thus the formation of leaks.

It is expedient to close the ends of the hollow fiber membranes before the potting step in order to prevent the potting material from permeating into the fibers. The fiber ends can be closed by processes known in the art, e.g., by melting or by means of an adhesive. In one embodiment of the process, the ends of the hollow fiber membranes are closed before the bundle 8 of hollow fiber membranes is introduced into the housing 4.

The ends of the hollow fiber membranes are subsequently opened, for instance by cutting part of the end wall means 9 with a blade. After the ends of the hollow fiber membranes have been opened, end caps featuring blood ports are mounted on the ends of the housing 4 of the dialyzer to close it. The end caps are bonded to the housing 4 by a suitable process, e.g., welding, heat-sealing, or by means of an adhesive, for instance, a polyurethane adhesive.

## Claims

1. A dialyzer housing (4) comprising
i) a cylindrical first section (1) having an inner diameter d₁;
ii) a cylindrical second section (2) coaxial with the first section (1) and having an inner diameter d₂ which is larger than d₁; wherein a plurality of openings (6) tapering from the outside towards the inside is provided in the wall of the second section (2); and
iii) a third section (3) joining the first section (1) and the second section (2), wherein a plurality of circumferential ridges (5) are provided on the inner surface of the third section (3), each circumferential ridge (5) having a tooth profile and pointing in the direction of the axis of the first section (1) away from the first section (1), with the faces of the circumferential ridge (5) forming an angle in the range of from 20° to 40, and the circumferential ridge (5) having an elevation relative to the inner wall surface of the third section (3) in the range of from 1 to 10 mm .

2. The dialyzer housing (4) of claim 1, wherein the height of the third section (3) is in the range of from 0.36*(d₂-d₁) to 2.75*(d₂-d₁).

3. The dialyzer housing (4) of claim 1 or 2, wherein the number of circumferential ridges (5) is 2 to 5.

4. The dialyzer housing (4) of claim 3, wherein the spacing of the tops of the individual circumferential ridges (5) is in the range of from 0.5 to 3 mm.

5. The dialyzer housing (4) of any one of claims 1 to 4, wherein the openings (6) have a diameter on the outside surface of the second section (2) in the range of from 1 to 4 mm.

6. The dialyzer housing (4) of any one of claims 1 to 5, wherein the first section (1) comprises fluid ports (7).

7. The dialyzer housing (4) of any one of claims 1 to 6, which is comprised of a polyolefin.

8. The dialyzer housing (4) of claim 7, wherein the polyolefin is polypropylene.

9. A capillary dialyzer comprising:
a) a housing (4) according to any one of claims 1 to 8, comprising second sections (2) and third sections (3) on each end of the first section (1), the housing (4) defining a longitudinally extending internal chamber including a first end and a second end;
b) a bundle (8) of semi-permeable hollow fiber membranes disposed within the internal chamber and extending longitudinally from the first end of the internal chamber to the second end of the internal chamber, the hollow fiber membranes each having an outer surface, and a first end and a second end corresponding to the first end and the second end of the internal chamber;
c) end wall means (9) supporting the first and second ends of the hollow fiber membranes within the internal chamber so as to sealingly separate the first ends and second ends of the hollow fiber membranes from the outer surfaces of the hollow fiber membranes between the first ends and second ends thereof, wherein the end wall means fill the third sections (3) and also fill the openings (6) of the second sections (2).

10. A process for producing a capillary dialyzer, comprising:
a) providing a housing (4) according to any one of claims 1 to 8, comprising second sections (2) and third sections (3) on each end of the first section (1), the housing (4) defining a longitudinally extending internal chamber including a first end and a second end;
b) introducing a bundle (8) of semi-permeable hollow fiber membranes having an outer surface, a first end and a second end into the internal chamber so that the bundle (8) extends longitudinally from the first end of the internal chamber to the second end of the internal chamber and the first ends and second ends of the hollow fiber membranes correspond to the first end and the second end of the internal chamber, respectively;
c) providing end wall means (9) supporting the first and second ends of the hollow fiber membranes within the internal chamber so as to sealingly separate the first ends and second ends of the hollow fiber membranes from the outer surface of the hollow fiber membranes between the first ends and second ends thereof, by introducing a potting material into the third sections (3) and second sections (2) of the housing (4) and filling the third sections (3) and the second sections (2) inclusive of the openings (6) in the wall of the second sections (2) with the potting material; and allowing the potting material to cure.

## Patentansprüche

1. Dialysatorengehäuse (4) umfassend
i) einen ersten zylindrischen Abschnitt (1) mit einem Innendurchmesser d₁;
ii) einen zweiten zylindrischen Abschnitt (2), der koaxial mit dem ersten Abschnitt (1) ist und einen Innendurchmesser d₂ aufweist, der größer ist als d₁; wobei sich in der Wand des zweiten Abschnitts (2) mehrere Öffnungen (6) befinden, die sich von der Außenseite zur Innenseite hin verjüngen; und
iii) einen dritten Abschnitt (3), der den ersten Abschnitt(1) und den zweiten Abschnitt (2) verbindet, wobei auf der inneren Oberfläche des dritten Abschnitts (3) mehrere umlaufende Rippen (5) angeordnet sind, wobei jede umlaufende Rippe (5) ein Zahnprofil aufweist und in Richtung der Achse des ersten Abschnitts (1) von dem ersten Abschnitt (1) weg zeigt, und die Flächen der umlaufenden Rippe (5) einen Winkel im Bereich von 20° bis 40° einschließen, und die umlaufende Rippe (5) eine Höhe, bezogen auf die Oberfläche der Innenwand des dritten Abschnitts (3), im Bereich von 1 bis 10 mm aufweist.

2. Dialysatorengehäuse (4) gemäß Anspruch 1, worin die Höhe des dritten Abschnitts (3) im Bereich von 0,36*(d₂-d₁) bis 2,75*(d₂-d₁) beträgt.

3. Dialysatorengehäuse (4) gemäß Anspruch 1 oder 2, worin die Anzahl der umlaufenden Rippen (5) 2 bis 5 ist.

4. Dialysatorengehäuse (4) gemäß Anspruch 3, worin der Abstand der Spitzen der einzelnen umlaufenden Rippen (5) im Bereich von 0,5 bis 3 mm beträgt.

5. Dialysatorengehäuse (4) gemäß einem der Ansprüche 1 bis 4, worin die Öffnungen (6) einen Durchmesser auf der Außenfläche des zweiten Abschnitts (2) im Bereich von 1 bis 4 mm aufweisen.

6. Dialysatorengehäuse (4) gemäß einem der Ansprüche 1 bis 5, worin der erste Abschnitt (1) Fluidanschlüsse (7) aufweist.

7. Dialysatorengehäuse (4) gemäß einem der Ansprüche 1 bis 6, das aus einem Polyolefin besteht.

8. Dialysatorengehäuse (4) gemäß Anspruch 7, worin das Polyolefin Polypropylen ist.

9. Hohlfaserdialysator umfassend:
a) ein Gehäuse (4) gemäß einem der Ansprüche 1 bis 8, mit zweiten Abschnitten (2) und dritten Abschnitten (3) an jedem Ende des ersten Abschnitts (1), wobei das Gehäuse (4) eine sich in Längsrichtung erstreckende Innenkammer mit einem ersten Ende und einem zweiten Ende definiert;
b) ein Bündel (8) semipermeabler Hohlfasermembranen, das in der Innenkammer angeordnet ist und sich in Längsrichtung von dem ersten Ende der Innenkammer bis zu dem zweiten Ende der Innenkammer erstreckt, wobei die Hohlfasermembranen jeweils eine Außenfläche und ein erstes Ende und ein zweites Ende aufweisen, die dem ersten Ende und dem zweiten Ende der Innenkammer entsprechen;
c) Endwandmittel (9), die die ersten und zweiten Enden der Hohlfasermembranen innerhalb der Innenkammer abstützen, um dadurch die ersten Enden und zweiten Enden der Hohlfasermembranen von den Außenflächen der Hohlfasermembranen zwischen deren ersten und zweiten Enden abdichtend zu separieren, wobei die Endwandmittel die dritten Abschnitte (3) ausfüllen und ebenfalls die Öffnungen (6) der zweiten Abschnitte (2) ausfüllen.

10. Verfahren zur Herstellung eines Hohlfaserdialysators, umfassend:
a) Bereitstellen eines Gehäuses (4) gemäß einem der Ansprüche 1 bis 8, mit zweiten Abschnitten (2) und dritten Abschnitten (3) an jedem Ende des ersten Abschnitts (1), wobei das Gehäuse (4) eine sich in Längsrichtung erstreckende Innenkammer mit einem ersten Ende und einem zweiten Ende definiert;
b) Einführen eines Bündels (8) semipermeabler Hohlfasermembranen mit einer Außenfläche, einem ersten Ende und einem zweiten Ende in die Innenkammer, so dass das Bündel (8) sich in Längsrichtung von dem ersten Ende der Innenkammer bis zu dem zweiten Ende der Innenkammer erstreckt und die ersten Enden der Hohlfasermembranen dem ersten Ende der Innenkammer entsprechen und die zweiten Enden der Hohlfasermembranen dem zweiten Ende der Innenkammer entsprechen;
c) Bereitstellen von Endwandmitteln (9) die die ersten und zweiten Enden der Hohlfasermembranen innerhalb der Innenkammer abstützen, um dadurch die ersten Enden und zweiten Enden der Hohlfasermembranen von den Außenflächen der Hohlfasermembranen zwischen deren ersten und zweiten Enden abdichtend zu separieren, durch Einführen eines Vergussmaterials in die dritten Abschnitte (3) und zweiten Abschnitte (2) des Gehäuses (4) und Befüllen der dritten Abschnitte (3) und der zweiten Abschnitte (2) einschließlich der Öffnungen (6) in der Wand der zweiten Abschnitte (2) mit dem Vergussmaterial; und Aushärten lassen des Vergussmaterials.

## Revendications

1. Boîtier (4) de dialyseur, comprenant
i) une première section cylindrique (1) ayant un diamètre interne d₁;
ii) une deuxième section cylindrique (2) coaxiale avec la première section (1) et ayant un diamètre interne d₂ qui est plus grand que d₁; une pluralité d'ouvertures (6) diminuant depuis l'extérieur vers l'intérieur étant prévue dans la paroi de la deuxième section (2); et
iii) une troisième section (3) joignant la première section (1) et la deuxième section (2), dans laquelle une pluralité de nervures circonférentielles (5) est prévue sur la surface interne de la troisième section (3), chaque nervure circonférentielle (5) ayant un profil de dent et pointant dans la direction de l'axe de la première section (1) à l'opposé de la première section (1), les faces de la nervure circonférentielle (5) formant un angle dans la plage de 20° à 40°, et la nervure circonférentielle (5) ayant une élévation par rapport à la surface de paroi interne de la troisième section (3) dans la plage de 1 à 10 mm.

2. Boîtier (4) de dialyseur selon la revendication 1, dans lequel la hauteur de la troisième section (3) est dans la plage de 0,36*(d₂-d₁) à 2,75*(d₂-d₁).

3. Boîtier (4) de dialyseur selon la revendication 1 ou 2, dans lequel le nombre de nervures circonférentielles (5) est de 2 à 5.

4. Boîtier (4) de dialyseur selon la revendication 3, dans lequel l'espacement des parties supérieures des nervures circonférentielles (5) individuelles est dans la plage de 0,5 à 3 mm.

5. Boîtier (4) de dialyseur selon l'une quelconque des revendications 1 à 4, dans lequel les ouvertures (6) ont un diamètre sur la surface extérieure de la deuxième section (2) dans la plage de 1 à 4 mm.

6. Boîtier (4) de dialyseur selon l'une quelconque des revendications 1 à 5, dans lequel la première section (1) comprend des orifices (7) de fluide.

7. Boîtier (4) de dialyseur selon l'une quelconque des revendications 1 à 6, qui est constitué d'une polyoléfine.

8. Boîtier (4) de dialyseur selon la revendication 7, dans lequel la polyoléfine est le polypropylène.

9. Dialyseur capillaire comprenant :
a) un boîtier (4) selon l'une quelconque des revendications 1 à 8, comprenant des deuxièmes sections (2) et des troisièmes sections (3) sur chaque extrémité de la première section (1), le boîtier (4) définissant une chambre interne s'étendant longitudinalement comprenant une première extrémité et une seconde extrémité;
b) un faisceau (8) de membranes en fibres creuses semi-perméables disposé à l'intérieur de la chambre interne et s'étendant longitudinalement depuis la première extrémité de la chambre interne vers la seconde extrémité de la chambre interne, les membranes en fibres creuses ayant chacune une surface externe, et une première extrémité et une seconde extrémité correspondant à la première extrémité et à la seconde extrémité de la chambre interne;
c) des moyens (9) de paroi d'extrémité supportant les première et seconde extrémités des membranes à fibres creuses à l'intérieur de la chambre interne de façon à séparer à étanchéité les premières extrémités et les secondes extrémités des membranes à fibres creuses des surfaces externes des membranes à fibres creuses entre les premières extrémités et les secondes extrémités associées, les moyens de paroi d'extrémité remplissant les troisièmes sections (3) et remplissant également les ouvertures (6) des deuxièmes sections (2).

10. Procédé de production d'un dialyseur capillaire, consistant à :
a) fournir un boîtier (4) selon l'une quelconque des revendications 1 à 8, comprenant des deuxièmes sections (2) et des troisièmes sections (3) sur chaque extrémité de la première section (1), le boîtier (4) définissant une chambre interne s'étendant longitudinalement comprenant une première extrémité et une seconde extrémité;
b) introduire un faisceau (8) de membranes en fibres creuses semi-perméables ayant une surface externe, une première extrémité et une seconde extrémité dans la chambre interne de sorte que le faisceau (8) s'étende longitudinalement depuis la première extrémité de la chambre interne vers la seconde extrémité de la chambre interne et que les premières extrémités et les secondes extrémités des membranes à fibres creuses correspondent à la première extrémité et à la seconde extrémité de la chambre interne, respectivement;
c) fournir des moyens (9) de paroi d'extrémité supportant les première et seconde extrémités des membranes à fibres creuses à l'intérieur de la chambre interne de façon à séparer à étanchéité les premières extrémités et les secondes extrémités des membranes à fibres creuses de la surface externe des membranes à fibres creuses entre les premières extrémités et les secondes extrémités associées, en introduisant un matériau d'enrobage dans les troisièmes sections (3) et les deuxièmes sections (2) du boîtier (4) et en remplissant les troisièmes sections (3) et des deuxièmes sections (2) y compris les ouvertures (6) dans la paroi des deuxièmes sections (2) avec le matériau d'enrobage; et en laissant durcir le matériau d'enrobage.
